# EUROPEAN PATENT APPLICATION

(11) **EP 2 883 538 A1**
(43) Date of publication of application: **17.06.2015**
(21) Application number: 13823181.6
(22) Date of filing: 25.07.2013
(51) Int. Cl.: A61K 8/49, A61K 8/34, A61K 8/73, A61K 9/08, A61K 31/513, A61K 47/10, A61K 47/38, A61P 17/14, A61Q 7/00

(54) **COMPOSITION FOR AGENT FOR EXTERNAL USE**

(30) Priority: 27.07.2012 JP 2012166882
(71) Applicant: Taisho Pharmaceutical Co., Ltd., Tokyo 170-8633 (JP)
(72) Inventor: IDA Tomoko, Tokyo 170-8633 (JP); IDEURA Saori, Tokyo 170-8633 (JP)
(74) Representative: Hoffmann Eitle
(86) International application number: PCT/JP2013/070144
(87) International publication number: WO 2014/017573

(57) **Abstract**

Upon attempting to design a pharmaceutical preparation by mixing minoxidil with a general thickening agent during a process for considering a minoxidil-containing composition for an agent for external use in which dripping is inhibited, it was discovered that the pharmaceutical preparation would have sediments or would cloud and that a sufficient viscosity of the pharmaceutical preparation could not be obtained. The minoxidil-containing composition for an agent for external use of the present invention is characterized by containing minoxidil (a), a hydroxypropyl cellulose and hypromellose (b), ethanol (c), and water (d). Said composition is clear and has a good feel upon use, and dripping of the composition is inhibited.

## Description

### Technical Field

The present invention relates a composition for agent for external use containing minoxidil as an active ingredient, and also relates to a minoxidil-containing composition for agent for external use, which is clear and viscous, is inhibited from dripping, and has a good comfort of use.

### Background Art

The chemical name of minoxidil is 6-(1-piperidinyl)-2,4-pyrimidinediamine-3-oxide, and the application of minoxidil as a hair restorer has been known (see Patent Literature 1). When externally applied, minoxidil has excellent hair-fostering and hair-growing effects. Thus, hair restorers containing minoxidil have been widely accepted. Currently, minoxidil-containing pharmaceutical preparations in lotion form are commercially available.

In the case of pharmaceutical preparations in lotion form, it is concerned that dripping may occur during use. Accordingly, there has been a demand for a dripless minoxidil-containing composition for agent for external use. However, in the actual situation, a minoxidil product with sufficiently inhibited dripping has not yet been provided. Incidentally, "dripping" herein means that in the case where a composition for agent for external use is applied to an affected part such as the scalp, the pharmaceutical solution runs off the application part, causing the deterioration of the comfort of use.

Generally, in order to provide a composition for agent for external use that is inhibited from dripping, it is possible to use a thickening agent.

### Citation List

### Patent Literature

Patent Literature 1: US 4139619

### Summary of Invention

### Technical Problem

Upon attempting to design a pharmaceutical preparation by mixing minoxidil with a general thickening agent during a process for considering a minoxidil-containing composition for agent for external use that is inhibited from dripping, the present inventors have discovered that such a pharmaceutical preparation would have sediments or would cloud and that a sufficient viscosity of the pharmaceutical preparation could not be obtained.

An object of the invention is to provide a minoxidil-containing composition for agent for external use that is inhibited from dripping, and also to provide a composition for agent for external use, which is a clear pharmaceutical preparation having a good comfort of use.

### Solution to Problem

The present inventors have conducted extensive research to solve the above problems. As a result, they have found that a composition for agent for external use containing minoxidil, hydroxypropyl cellulose, hypromellose, ethanol, and water is clear and viscous, is free from dripping, has moderate adhesion at an affected part, and has a good comfort of use. The present invention has thus been accomplished based on this finding.

That is, the present invention provides:
(1) A composition for agent for external use, comprising:
   (a) minoxidil,
   (b) hydroxypropyl cellulose and hypromellose,
   (c) ethanol, and
   (d) water;
(2) The composition for agent for external use according to (1), having a hydroxypropyl cellulose content of 0.05 to 2.5 w/v%;
(3) The composition for agent for external use according to (1) or (2), having a hydroxypropyl cellulose content of 0.1 to 2 w/v%;
(4) The composition for agent for external use according to any one of (1) to (3), having a viscosity of 30 to 250 mPa·s at 25°C;
(5) The composition for agent for external use according to any one of (1) to (4), further comprising an acid;
(6) The composition for agent for external use according to any one of (1) to (5), wherein the acid is at least one acid selected from the group consisting of citric acid, hydrochloric acid, lactic acid, phosphoric acid, tartaric acid, and gluconic acid;
(7) The composition for agent for external use according to any one of (1) to (6), having a pH of 5.0 to 8.5;
(8) The composition for agent for external use according to any one of (1) to (7), having a pH of 5.5 to 7.0;
(9) The composition for agent for external use according to any one of (1) to (8), having an ethanol content of 25 to 70 w/v%;
(10) The composition for agent for external use according to any one of (1) to (9), having an ethanol content of 50 to 70 w/v%;
(11) The composition for agent for external use according to any one of (1) to (10), having a minoxidil content of 1 to 10 w/v%;
(12) The composition for agent for external use according to any one of (1) to (11), further comprising a polyalcohol;
(13) The composition for agent for external use according to (12), wherein the polyalcohol is at least one member selected from the group consisting of 1,3-butylene glycol, propylene glycol, glycerin, dipropylene glycol, Macrogol 400, and Macrogol 600;
(14) The composition for agent for external use according to (12), wherein the polyalcohol is 1,3-butylene glycol; and
(15) The composition for agent for external use according to (1) to (14), having a dosage form of gel, lotion, or liquid.

### Advantageous Effects of Invention

The present invention enables the provision of a minoxidil-containing composition for agent for external use, which is clear and viscous, is inhibited from dripping, and has a good comfort of use.

### Description of Embodiments

The present invention will be described in detail hereinafter.

As the minoxidil to be used in the present invention, general pharmaceutical quality ones can be suitably used. The amount of minoxidil incorporated ranges 1 to 10 w/v%, preferably 3 to 8 w/v%, and more preferably 5 to 8 w/v% in the composition for agent for external use.

In the composition for agent for external use of the present invention, hydroxypropyl cellulose and hypromellose are incorporated as thickening agents.

With respect to the hydroxypropyl cellulose, it is preferable for it to have a viscosity of its 2 w/v% aqueous solution at 20°C (measured according to The Japanese Pharmacopoeia, 14th Edition, The Second Method (rotational viscometer)) of more than 150 mPa·s and 4,000 mPa·s or less. As the hydroxypropyl cellulose to be used, general pharmaceutical quality ones can be suitably used. The hydroxypropyl cellulose may be a commercially available product. For example, it is possible to use NISSO HPC-H (Nippon Soda Co., Ltd.), NISSO HPC-M (Nippon Soda Co., Ltd.), etc. In addition, the amount of hydroxypropyl cellulose incorporated ranges preferably 0.05 to 2.5 w/v%, and more preferably 0.1 to 2 w/v%, in the composition for agent for external use of the present invention.

With respect to the hypromellose, it is preferable for it to have an viscosity of its 2 w/v% aqueous solution at 20°C (measured according to The Japanese Pharmacopoeia, 14th Edition, The Second Method (rotational viscometer)) of more than 7,500 mPa·s and 14,000 mPa·s or less. As the hypromellose to be used, general pharmaceutical quality ones can be suitably used. The hypromellose may be a commercially available product. For example, it is possible to use METOLOSE 60SH-10000 (Shin-Etsu Chemical Co., Ltd.), etc. In addition, the amount of hypromellose incorporated ranges preferably 0.01 to 1 w/v%, and more preferably 0.01 to 0.5 w/v%, in the composition for agent for external use of the present invention.

It is preferable that the viscosity of the composition for agent for external use of the present invention ranges 30 to 250 mPa·s at 25°C. The composition for agent for external use having a viscosity within the above range can be obtained by suitably selecting the kind and amount of hydroxypropyl cellulose and hypromellose to be used mainly. Therefore, the ratio between the contents of hydroxypropyl cellulose and hypromellose is not particularly limited. In addition, it is necessary that the composition for agent for external use of the present invention contains both hydroxypropyl cellulose and hypromellose. For example, even if a viscosity of 30 mPa·s or more is achieved by hydroxypropyl cellulose alone without using hypromellose, dripping cannot necessarily be ameliorated. Incidentally, the viscosity of the composition for agent for external use of the present invention is measured using an oscillatory viscometer. In this application, the viscosity at 25°C is determined using VISCOMATE VM-100A (Yamaichi Electronics, Inc.), and the conditions, such as the probe to be used, are selected according to its operation manual.

The composition for agent for external use of the present invention contains hydroxypropyl cellulose and hypromellose as thickening agents. Attributable to this, it is now possible to produce a clear, minoxidil-containing composition for agent for external use with a good comfort of use, which is inhibited from dripping, and, moreover, is free from causing undesirable stickiness or caking when applied.

In the composition for agent for external use of the present invention, an acid can be used to adjust the pH. Examples of acids include citric acid, hydrochloric acid, lactic acid, phosphoric acid, tartaric acid, and gluconic acid. For each, pharmaceutical quality products can be suitably used. In addition, the pH of the composition for agent for external use of the present invention ranges preferably 5 to 8.5, and more preferably 5.5 to 7.

The amount of ethanol incorporated in the present invention ranges preferably 25 to 70 w/v% in the composition for agent for external use. When ethanol is incorporated into a composition for agent for external use in a large amount, thickening agents that can be incorporated while keeping the clearness of the composition are limited. Therefore, it would be an outstanding advantage of the minoxidil-containing composition for agent for external use of the present invention that the present invention may be implemented with an amount of ethanol incorporated being within the range of 50 w/v% or more.

The amount of water incorporated in the present invention ranges preferably 5 to 30 w/v% in the composition for agent for external use. As each of the ethanol and water to be used, general pharmaceutical quality ones can be suitably used.

The composition for agent for external use of the present invention may contain a polyalcohol. Examples of usable polyalcohols include 1,3-butylene glycol, propylene glycol, glycerin, dipropylene glycol, Macrogol 400, and Macrogol 600. In particular, 1,3-butylene glycol is preferable. The amount of polyalcohol incorporated ranges preferably 1 to 30 w/v% in the composition for agent for external use, and more preferably 5 to 20 w/v% in the composition for agent for external use. As the polyalcohol to be used, general pharmaceutical quality ones can be suitably used.

The dosage form of the minoxidil-containing composition for agent for external use of the present invention is not particularly limited. The dosage form is preferably a gel, a lotion, or a liquid.

Further, components that can optionally be incorporated into the composition for agent for external use of the present invention include those which are selected from the group consisting of menthol, tocopherol acetate, pyridoxine hydrochloride, and pantothenyl ethyl ether (hereinafter referred to as "optional components"). The amount thereof incorporated is not particularly limited, and can be determined considering the comfort of use, the stability of minoxidil, etc. As each of the optional components to be used, general pharmaceutical quality ones can be suitably used.

In the composition for agent for external use of the present invention, in addition to the components mentioned above, various components generally used for agents for external use can be incorporated without interfering with the advantageous effects of the present invention. Examples thereof include components that are generally used, such as excipients, hair-fostering components (6-benzyl aminopurine, adenosine, pentadecanoic acid glyceride, Polygonum multiflorum, Panax japonicus, etc.), vasodilators (carpronium chloride, benzyl nicotinate, swertia herb extract, Panax schinseng extract, capsicum tincture, etc.), antihistamines (diphenhydramine hydrochloride, isothipendyl hydrochloride, etc.), anti-inflammatory agents (guaiazulene, etc.), keratolytic drugs (urea, salicylic acid, etc.), disinfectants (chlorhexidine glyconate, isopropylmethylphenol, quaternary ammonium salts, piroctone olamine, etc.), moisturizers (hyaluronic acid or a salt thereof, chondroitin sulfate, etc.), extracts of various animals and plants (Taxus cuspidata, moutan bark, liquorice, St. John's wort, aconite, loquat, Artemisia capillaris, comfrey, angelica, crocus, gardenia fruit, rosemary, sage, Saussurea root, Aristolochia root, hop, placenta, etc.), vitamins (retinol acetate, ascorbic acid, thiamine nitrate, cyanocobalamine, biotin, etc.), antioxidants (dibutylhydroxytoluene, sodium pyrosulfite, tocopherol, sodium edetate, ascorbic acid, isopropyl gallate, etc.), solubilizers (diisopropyl adipate, isopropyl myristate, various vegetable oils, various animal oils, hydrocarbons, etc.), metabolism activators (panthenol, etc.), adhesives, perfumes, refreshing agents (mentha oil, camphor, etc.), and dyes.

For the preparation of the composition for agent for external use of the present invention, for example, hydroxypropyl cellulose or hypromellose is swollen using purified water or ethanol, then minoxidil is added thereto, and further the above components are incorporated therein to prepare the composition. When the obtained composition for agent for external use has low viscosity, it can be provided as a liquid or a lotion. In the case of high viscosity, it is provided as a gel.

The composition for agent for external use of the present invention thus obtained can be used as an agent for hair, a pharmaceutical preparation for skin application, etc.

Hereinafter, test examples, examples, and comparative examples will be given to describe the present invention in further detail. However, the present invention is not limited to these examples, etc.

### Test Example 1 (Influence of the Variation of Thickening Agent)

According to the formulation shown in Table 1 below, the components were weighed. Each of the thickening agents was dispersed and swollen in water, ethanol or 1,3-butylene glycol, then dibutylhydroxytoluene, minoxidil and phosphoric acid were added thereto, and the rest of the ethanol and purified water were further added thereto to make the total volume of 100mL, to provide pharmaceutical preparations of Reference Examples 1 to 11, respectively. The external appearance of the provided pharmaceutical preparations was visually observed and evaluated according to the following criteria. The results are shown in Table 2.

### [Criteria]

### External Appearance

○: The formation of sediments, clouding, or separation was not seen.
×: The formation of sediments, clouding, or separation was seen.

**[Table 1]**

| | | Reference Example 1 | Reference Example 2 | Reference Example 3 | Reference Example 4 | Reference Example 5 | Reference Example 6 | Reference Example 7 | Reference Example 8 | Reference Example 9 | Reference Example 10 | Reference Example 11 |
|---|---|---|---|---|---|---|---|---|---|---|---|---|
| Minoxidil | | 5g | 5g | 5g | 5g | 5g | 5g | 5g | 5g | 5g | 5g | 5g |
| Phosphoric acid | | 0.847g | 0.847g | 0.847g | 0.847g | 0.847g | 0.847g | 0.847g | 0.847g | 0.847g | 0.847g | 0.847g |
| Thickening agent | Hydroxypropyl cellulose | 1.2g | | | | | | | | | | |
| | Hypromellose | | 1g | | | | | | | | | |
| | Carboxyvinyl polymer | | | 1g | | | | | | | | |
| | Xanthan gum | | | | 0.3g | | | | | | | |
| | Hydroxyethyl cellulose | | | | | 1g | | | | | | |
| | Polyvinyl alcohol (partially saponified) | | | | | | 1g | | | | | |
| | Methyl vinyl ether-maleic anhydride copolymer | | | | | | | 1g | | | | |
| | Carmellose sodium | | | | | | | | 1g | | | |
| | Pectin | | | | | | | | | 1g | | |
| | Ethyl cellulose 100 | | | | | | | | | | 0.2g | |
| | Polyvinyl pyrrolidone K90 | | | | | | | | | | | 1g |
| Dibutylhydroxytoluene | | 0.05g | 0.05g | 0.05g | 0.05g | 0.05g | 0.05g | 0.05g | 0.05g | 0.05g | 0.05g | 0.05g |
| 1,3-Butylene glycol | | 10g | 10g | 10g | 10g | 10g | 10g | 10g | 10g | 10g | 10g | 10g |
| Ethanol (95) | | 60g | 60g | 60g | 60g | 60g | 60g | 60g | 60g | 60g | 60g | 60g |
| Purified water | | 100 mL in total | 100 mL in total | 100 mL in total | 100 mL in total | 100 mL in total | 100 mL in total | 100 mL in total | 100 mL in total | 100 mL in total | 100 mL in total | 100 mL in total |

**[Table 2]**

| | External Appearance |
|---|---|
| Reference Example 1 | ○ |
| Reference Example 2 | ○ |
| Comparative Example 3 | × |
| Comparative Example 4 | × |
| Comparative Example 5 | × |
| Comparative Example 6 | × |
| Comparative Example 7 | × |
| Reference Example 8 | × |
| Reference Example 9 | × |
| Reference Example 10 | ○ |
| Reference Example 11 | ○ |

The above results show that the pharmaceutical preparations of Reference Examples 1, 2, 10, and 11 using hydroxypropyl cellulose, hypromellose, ethyl cellulose, and polyvinyl pyrrolidone, respectively, were free from the formation of sediments or clouding. However, with respect to the pharmaceutical preparations of Reference Examples 3 to 9 using carboxyvinyl polymer, xanthan gum, hydroxyethyl cellulose, polyvinyl alcohol, methyl vinyl ether-maleic anhydride copolymer, carmellose sodium, or pectin, respectively, the formation of sediments, clouding, or the like occurred during preparation, and it was difficult to prepare a pharmaceutical preparation with a clear external appearance.

According to the formulation shown in Tables 3 and 4 below, the components were weighed. Each of the thickening agents were dispersed and swollen in water, ethanol, or a polyalcohol, then minoxidil and an acid were added thereto, and the rest of the ethanol and purified water were further added thereto to make the total amount of 100 mL, to provide pharmaceutical preparations of Examples 1 to 11 and Comparative Examples 1 to 10. The provided pharmaceutical preparations were each placed in a glass bottle.

**[Table 3]**

| | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| Minoxidil | 5g | 5g | 5g | 5g | 5g | 5g | 3g | 8g | 5g | 3g | 5g |
| Phosphoric acid | 0.847g | 0.847g | 0.847g | 0.847g | 0.847g | 0.847g | - | - | 0.847g | 0.339g | 0.847g |
| Citric acid | - | - | - | - | - | - | - | - | - | - | - |
| Lactic acid | - | - | - | - | - | - | - | - | - | - | - |
| Hydroxypropyl cellulose (HPC-M) | 0.5g | 0.8g | 0.1g | 1.0g | 2g | 2g | 0.8g | 0.8g | 0.8g | 2g | 0.8g |
| Hypromellose (METOLOSE 60SH-10000) | 0.5g | 0.2g | 0.5g | 0.2g | 0.01g | 0.3g | 0.2g | 0.2g | 0.2g | 0.01g | 0.2g |
| 1,3-Butylene glycol | 10g | 10g | 10g | 10g | 10g | 10g | 8g | - | 10g | 7g | - |
| Propylene glycol | - | - | - | - | - | - | - | 10g | - | - | - |
| Ethanol (95) | 60g | 60g | 60g | 60g | 60g | 60g | 60g | 60g | 50g | 70g | 60g |
| Purified water | 100 mL in total | 100 mL in total | 100 mL in total | 100 mL in total | 100 mL in total | 100 mL in total | 100 mL in total | 100 mL in total | 100 mL in total | 100 mL in total | 100 mL in total |
| pH | 6.13 | 6.13 | 6.12 | 6.15 | 6.16 | 6.13 | 6.47 | 5.93 | 5.82 | 6.47 | 5.86 |
| Viscosity | 60.4mPa·s | 47.0mPa·s | 30.8mPa·s | 58.7mPa·s | 124mPa·s | 214mPa·s | 40.5mPa·s | 44.9mPa·s | 49.1mPa·s | 100mPa·s | 41.7mPa·s |

**[Table 4]**

| | Comparative Example 1 | Comparative Example 2 | Comparative Example 3 | Comparative Example 4 | Comparative Example 5 | Comparative Example 6 | Comparative Example 7 | Comparative Examples 8 | Comparative Example 9 | Comparative Example 10 |
|---|---|---|---|---|---|---|---|---|---|---|
| Minoxidil | 5g | 5g | 5g | 5g | 5g | 5g | 5g | 5g | 5g | 5g |
| Phosphoric acid | 0.847g | 0.847g | 0.847g | 0.847g | 0.847g | 0.847 g | 0.847g | 0.847g | 0.647g | 0.847g |
| Hydroxypropyl cellulose (HPC-M) | 0.1g | 0.5g | 1g | 5g | - | - | - | - | 0.5g | 0.5g |
| Hypromellose (METOLOSE 60SH-10000) | - | - | - | - | 0.05g | 0.1g | 0. 5g | 1g | - | - |
| Ethyl cellulose 100 | - | - | - | - | - | - | - | - | 1s | - |
| Polyvinyl pyrrolidone K90 | - | - | - | - | - | - | - | - | - | 1g |
| 1,3-Butylene glycol | 10g | 10g | 10g | 10g | 10g | 10g | 10g | 10g | 10g | 10g |
| Ethanol (95) | 60g | 60g | 60g | 60g | 60g | 60g | 60g | 60g | 60g | 60g |
| Purified water | 100 mL in total | 100 mL in total | 100 mL in total | 100 mL in total | 100 mL in total | 100 mL in total | 100 mL in total | 100 mL in total | 100 mL i, total | 100 mL in total |
| pH | 6.13 | 6.13 | 6.14 | 6.20 | 6.08 | 6.11 | 6.11 | 6.07 | 6.05 | 6.11 |
| Viscosity | 4.43mPa·s | 14.6mPa·s | 38.5mPa·s | 600mPa·s | 4.14mPa·s | 5.47mPa·s | 28.7mPa·s | 90.3mPa·s | 39.8mPa·s | 30.9mPa·s |

### Test Example 2 (Dripping Test)

An alcohol test paper was placed on a flat plate at an angle of 20°, and 100 µL of each of the pharmaceutical preparations of Examples 1 to 6 and 11 and Comparative Examples 1 to 10 was discharged onto the alcohol test paper at an angle of 90°. Thirty (30) seconds later, the dripping distance on the alcohol test paper was measured and evaluated according to the following evaluation criteria.

### [Evaluation Criteria]

○: The dripping distance after 30 seconds is 50 mm or less.
×: The dripping distance after 30 seconds is more than 50 mm.

### Test Example 3 (Comfort of Use Test)

The comfort of use of the pharmaceutical preparations was evaluated by three special panelists. A sensory evaluation test was performed by the following scoring method: the pharmaceutical preparations of Examples 1 to 6 and 11 and Comparative Examples 1 to 10 were applied to the inside of the upper arm of three special panelists, and each panelist evaluated the preparations for "stickiness", "caking", and "spread". Incidentally, "caking" herein refers to the phenomenon of appearing of the composition for agent for external use as a white powdery mass at or after the application of the composition to the scalp. As the evaluation method, five ratings from 1 to 5 were set for each evaluation item, and the panelists gave any one of 1 to 5. Evaluation was made according to the following evaluation criteria.

### [Evaluation Items]

### 1) Stickiness

No stickiness is felt: Score 5
Slight stickiness is felt: Score 4
Stickiness is felt: Score 3
Slightly strong stickiness is felt: Score 2
Full stickiness is felt: Score 1

### 2) Caking

No caking: Score 5
Caking occurred (slight caking): Score 4
Caking occurred (some caking): Score 3
Caking occurred (slightly significant caking): Score 2
Caking occurred (significant caking): Score 1

### 3) Spread

Easy to spread (very easy): Score 5
Easy to spread (easy): Score 4
Easy to spread (slightly easy): Score 3
Hard to spread (slightly hard): Score 2
Hard to spread (very hard): Score 1

### [Evaluation Criteria]

○: The average of the scores for each evaluation item is 3.5 or more.
×: The average of the scores for each evaluation item is less than 3.5.

Table 5 shows the results of the dripping test and the comfort of use test on Examples 1 to 6 and 11 and Comparative Examples 1 to 10, as well as their overall evaluation.

**[Table 5]**

| | Dripping test | Comfort-of-use test | | | Overall evaluation |
|---|---|---|---|---|---|
| | | Stickiness | Caking | Spread | |
| Example 1 | ○ | ○ | ○ | ○ | ○ |
| Example 2 | ○ | ○ | ○ | ○ | ○ |
| Example 3 | ○ | ○ | ○ | ○ | ○ |
| Example 4 | ○ | ○ | ○ | ○ | ○ |
| Example 5 | ○ | ○ | ○ | ○ | ○ |
| Example 6 | ○ | ○ | ○ | ○ | ○ |
| Example 11 | ○ | ○ | ○ | ○ | ○ |
| Comparative Example 1 | × | ○ | ○ | ○ | × |
| Comparative Example 2 | × | ○ | ○ | ○ | × |
| Comparative Example 3 | × | ○ | ○ | ○ | × |
| Comparative Example 4 | ○ | × | × | × | × |
| Comparative Example 5 | × | ○ | ○ | ○ | × |
| Comparative Example 6 | × | ○ | ○ | ○ | × |
| Comparative Example 7 | × | ○ | ○ | ○ | × |
| Comparative Example 8 | ○ | × | × | ○ | × |
| Comparative Example 9 | × | ○ | ○ | ○ | × |
| Comparative Example 10 | × | ○ | ○ | ○ | × |

The results from Test Examples 2 and 3 showed that no minoxidil-containing composition for agent for external use that is satisfactory in terms of both the dripping and the comfort of use could be achieved by using hydroxypropyl cellulose or hypromellose each alone as a thickening agent; however, incorporating hydroxypropyl cellulose and hypromellose in combination could produce a pharmaceutical preparation that is prevented from dripping, is free from causing stickiness and caking, is easy to spread, and has a good comfort of use. Moreover, it was shown that dripping was effectively inhibited in Examples 1 and 2 than Comparative Example 3 containing the same amount of thickening agent. Further, it was shown that dripping is effectively inhibited in Example 3, in spite of the fact that the viscosity is lower than Comparative Example 3. Furthermore, it was shown that the preparations in Examples 1 and 2 are superior in terms of the comfort of use evaluated by being free from stickiness and caking, than Comparative Example 8 containing the same amount of thickening agent.

### Industrial Applicability

According to the present invention, a minoxidil-containing composition for agent for external use that is inhibited from dripping and has a good comfort of use can be provided used as an agent for hair, a pharmaceutical preparation for skin application, etc.

Hereinafter, test examples, examples, and comparative examples will be given to describe the present invention in further detail. However, the present invention is not limited to these examples, etc.

### Test Example 1 (Influence of the Variation of Thickening Agent)

According to the formulation shown in Table 1 below, the components were weighed. Each of the thickening agents was dispersed and swollen in water, ethanol or 1,3-butylene glycol, then dibutylhydroxytoluene, minoxidil and phosphoric acid were added thereto, and the rest of the ethanol and purified water were further added thereto to make the total volume of 100mL, to provide pharmaceutical preparations of Reference Examples 1 to 11, respectively. The external appearance of the provided pharmaceutical preparations was visually observed and evaluated according to the following criteria. The results are shown in Table 2.

### [Criteria]

### External Appearance

○: The formation of sediments, clouding, or separation was not seen.
×: The formation of sediments, clouding, or separation was seen.

**[Table 1]**

| | | Reference Example 1 | Reference Example 2 | Reference Example 3 | Reference Example 4 | Reference Example 5 | Reference Example 6 | Reference Example 7 | Reference Example 8 | Reference Example 9 | Reference Example 10 | Reference Example 11 |
|---|---|---|---|---|---|---|---|---|---|---|---|---|
| Minoxidil | | 5g | 5g | 5g | 5g | 5g | 5g | 5g | 5g | 5g | 5g | 5g |
| Phosphoric acid | | 0.847 g | 0.847 g | 0.847 g | 0.847 g | 0.847 g | 0.847 g | 0.847 g | 0.847 g | 0.847 g | 0.847 g | 0.847 g |
| Thickening agent | Hydroxypropyl cellulose | 1.2 g | | | | | | | | | | |
| | Hypromellose | | 1g | | | | | | | | | |
| | Carboxyvinyl polymer | | | 1g | | | | | | | | |
| | Xanthan gum | | | | 0.3 g | | | | | | | |
| | Hydroxyethyl cellulose | | | | | 1 g | | | | | | |
| | Polyvinyl alcohol (partially saponified) | | | | | | 1g | | | | | |
| | Methyl vinyl ether-maleic anhydride copolymer | | | | | | | 1g | | | | |
| | Carmellose sodium | | | | | | | | 1g | | | |
| | Pectin | | | | | | | | | 1g | | |
| | Ethyl cellulose 100 | | | | | | | | | | 0.2 g | |
| | Polyvinyl pyrrolidone K90 | | | | | | | | | | | 1g |
| Dibutylhydroxytoluene | | 0.05 g | 0.05 g | 0.05 g | 0.05 g | 0.05 g | 0.05 g | 0.05 g | 0.05 g | 0.05 g | 0.05 g | 0.05 g |
| 1,3-Butylene glycol | | 10 g | 10 g | 10 g | 10 g | 10 g | 10 g | 10 g | 10g | 10 g | 10 g | 10g |
| Ethanol (95) | | 60 g | 60 g | 60 g | 60 g | 60 g | 60 g | 60 g | 60 g | 60 g | 60 g | 60 g |
| Purified water | | 100 mL in total | 100 mL in total | 100 mL in total | 100 mL in total | 100 mL in total | 100 mL in total | 100 mL in total | 100 mL in total | 100 mL in total | 100 mL in total | 100 mL in total |

**[Table 2]**

| | External Appearance |
|---|---|
| Reference Example 1 | ○ |
| Reference Example 2 | ○ |
| Comparative Example 3 | × |
| Comparative Example 4 | × |
| Comparative Example 5 | × |
| Comparative Example 6 | × |
| Comparative Example 7 | × |
| Reference Example 8 | × |
| Reference Example 9 | × |
| Reference Example 10 | ○ |
| Reference Example 11 | ○ |

The above results show that the pharmaceutical preparations of Reference Examples 1, 2, 10, and 11 using hydroxypropyl cellulose, hypromellose, ethyl cellulose, and polyvinyl pyrrolidone, respectively, were free from the formation of sediments or clouding. However, with respect to the pharmaceutical preparations of Reference Examples 3 to 9 using carboxyvinyl polymer, xanthan gum, hydroxyethyl cellulose, polyvinyl alcohol, methyl vinyl ether-maleic anhydride copolymer, carmellose sodium, or pectin, respectively, the formation of sediments, clouding, or the like occurred during preparation, and it was difficult to prepare a pharmaceutical preparation with a clear external appearance.

According to the formulation shown in Tables 3 and 4 below, the components were weighed. Each of the thickening agents were dispersed and swollen in water, ethanol, or a polyalcohol, then minoxidil and an acid were added thereto, and the rest of the ethanol and purified water were further added thereto to make the total amount of 100 mL, to provide pharmaceutical preparations of Examples 1 to 11 and Comparative Examples 1 to 10. The provided pharmaceutical preparations were each placed in a glass bottle.

**[Table 3]**

| | Example 1 | Example 2 | Example 3 | Example 4 | Example 5 | Example 6 | Example7 | Example 8 | Example 9 | Example 10 | Example 11 |
|---|---|---|---|---|---|---|---|---|---|---|---|
| Minoxidil | 5g | 5g | 5g | 5g | 5g | 5g | 3g | 8g | 5g | 3g | 5g |
| Phosphoric acid | 0.847 g | 0.847 g | 0.847 g | 0.847 g | 0.847 g | 0.847 g | --- | --- | 0.847 g | 0.339 g | 0.847 g |
| Citric acid | --- | --- | --- | --- | --- | --- | 0.2 g | --- | --- | --- | --- |
| Lactic acid | --- | --- | --- | --- | --- | --- | | 2g | --- | --- | --- |
| Hydroxypropyl cellulose (HPC-M) | 0.5 g | 0.8 g | 0.1 g | 1.0 g | 2g | 2 g | 0.8 g | 0.8 g | 0.8 g | 2 g | 0.8 g |
| Hypromellose (METOLOSE 60SH-10000) | 0.5 g | 0.2 g | 0.5 g | 0.2 g | 0.01 g | 0.3 g | 0.2 g | 0.2 g | 0.2 g | 0.01 g | 0.2 g |
| 1,3-Butylene glycol | 10 g | 10 g | 10g | 10 g | 10g | 10 g | 8g | --- | 10g | 7 g | --- |
| Propylene glycol | --- | --- | --- | --- | --- | --- | --- | 10g | --- | --- | --- |
| Ethanol (95) | 60 g | 60 g | 60 g | 60 g | 60 g | 60 g | 60 g | 60 g | 50 g | 70 g | 60 g |
| Purified water | 100 mL in total | 100 mL in total | 100 mL in total | 100 mL in total | 100 mL in total | 100 mL in total | 100 mL in total | 100 mL in total | 100 mL in total | 100 mL in total | 100 mL in total |
| pH | 6.13 | 6.13 | 6.12 | 6.15 | 6.16 | 6.13 | 6.47 | 5.93 | 5.82 | 6.47 | 5.86 |
| Viscosity | 60.4mPa·s | 47.0mPa·s | 30.8mPa·s | 58.7mPa·s | 124mPa·s | 214mPa·s | 40.5mPa·s | 44.9mPa·s | 49.1mPa·s | 100 mPa·s | 41.7mPa·s |

**[Table 4]**

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|
| Minoxidil | 5g | 5g | 5g | 5g | 5g | 5g | 5g | 5g | 5g | 5g g |
| Phosphoric acid | 0.847 g | 0.847 g | 0.847 g | 0.847 g | 0.847 g | 0.847 g | 0.847 g | 0.847 g | 0.847 g | 0.847 g |
| Hydroxypropyl cellulose (HPC-M) | 0.1 g | 0.5 g | 1 g | 5 g | --- | --- | --- | --- | 0.5 g | 0.5 g |
| Hypromellose (METOLOSE 60SH-10000) | --- | --- | --- | --- | 0.05 g | 0.1 g | 0.5 g | 1 g | --- | --- |
| Ethyl cellulose 100 | --- | --- | --- | --- | --- | --- | --- | --- | 1 g | --- |
| Polyvinyl pyrrolidone K90 | --- | --- | --- | --- | --- | --- | --- | --- | --- | 1 g |
| 1,3-Butylene glycol | 10 g | 10 g | 10 g | 10 g | 10 g | 10 g | 10 g | 10 g | 10 g | 10 g |
| Ethanol (95) | 60 g | 60 g | 60 g | 60 g | 60 g | 60 g | 60 g | 60 g | 60 g | 60 g |
| Purified water | 100 mL in total | 100 mL in total | 100 mL in total | 100 mL in total | 100 mL in total | 100 mL in total | 100 mL in total | 100 mL in total | 100 mL in total | 100 mL in total |
| pH | 6.13 | 6.13 | 6.14 | 6.20 | 6.08 | 6.11 | 6.11 | 6.07 | 6.05 | 6.11 |
| Viscosity | 4.43mPa·s | 14.6mPa·s | 38.5mPa·s | 600mPa·s | 4.14mPa·s | 5.47mPa·s | 28.7mPa·s | 90.3mPa·s | 39.8mPa·s | 30.9mPa·s |

### Test Example 2 (Dripping Test)

An alcohol test paper was placed on a flat plate at an angle of 20°, and 100 µL of each of the pharmaceutical preparations of Examples 1 to 6 and 11 and Comparative Examples 1 to 10 was discharged onto the alcohol test paper at an angle of 90°. Thirty (30) seconds later, the dripping distance on the alcohol test paper was measured and evaluated according to the following evaluation criteria.

### [Evaluation Criteria]

○: The dripping distance after 30 seconds is 50 mm or less.
×: The dripping distance after 30 seconds is more than 50 mm.

### Test Example 3 (Comfort of Use Test)

The comfort of use of the pharmaceutical preparations was evaluated by three special panelists. A sensory evaluation test was performed by the following scoring method: the pharmaceutical preparations of Examples 1 to 6 and 11 and Comparative Examples 1 to 10 were applied to the inside of the upper arm of three special panelists, and each panelist evaluated the preparations for "stickiness", "caking", and "spread". Incidentally, "caking" herein refers to the phenomenon of appearing of the composition for agent for external use as a white powdery mass at or after the application of the composition to the scalp. As the evaluation method, five

## Claims

1. A composition for agent for external use, comprising:
(a) minoxidil,
(b) hydroxypropyl cellulose and hypromellose,
(c) ethanol, and
(d) water.

2. The composition for agent for external use according to claim 1, having a hydroxypropyl cellulose content of 0.05 to 2.5 w/v%.

3. The composition for agent for external use according to claim 1 or 2, having a hydroxypropyl cellulose content of 0.1 to 2 w/v%.

4. The composition for agent for external use according to any one of claims 1 to 3, having a viscosity of 30 to 250 mPa·s at 25°C.

5. The composition for agent for external use according to any one of claims 1 to 4, further comprising an acid.

6. The composition for agent for external use according to claim 5, wherein the acid is at least one acid selected from the group consisting of citric acid, hydrochloric acid, lactic acid, phosphoric acid, tartaric acid, and gluconic acid.

7. The composition for agent for external use according to any one of claims 1 to 6, having a pH of 5.0 to 8.5.

8. The composition for agent for external use according to any one of claims 1 to 7, having a pH of 5.5 to 7.0.

9. The composition for agent for external use according to any one of claims 1 to 8, having an ethanol content of 25 to 70 w/v%.

10. The composition for agent for external use according to any one of claims 1 to 9, having an ethanol content of 50 to 70 w/v%.

11. The composition for agent for external use according to any one of claims 1 to 10, having a minoxidil content of 1 to 10 w/v%.

12. The composition for agent for external use according to any one of claims 1 to 11, further comprising a polyalcohol.

13. The composition for external use according to claim 12, wherein the polyalcohol is at least one member selected from the group consisting of 1,3-butylene glycol, propylene glycol, glycerin, dipropylene glycol, Macrogol 400, and Macrogol 600.

14. The composition for agent for external use according to claim 12, wherein the polyalcohol is 1,3-butylene glycol.

15. The composition for agent for external use according to claim 1 to 14, having a dosage form of gel, lotion, or liquid.
